Europäisches Patentamt

European Patent Office

Office européen des brevets

(11) Publication number: **0 451 484 A1**

# EUROPEAN PATENT APPLICATION

(21) Application number: **91102994.0**

(22) Date of filing: **28.02.91**

(51) Int. Cl.⁵: **A61K 31/195**, ///(A61K31/195, 31:14)

(30) Priority: **28.02.90 US 506967**

(43) Date of publication of application:
**16.10.91 Bulletin 91/42**

(84) Designated Contracting States:
**AT BE CH DE DK ES FR GB GR IT LI LU NL SE**

(71) Applicant: **DU PONT MERCK PHARMACEUTICAL COMPANY**

**Wilmington, Delaware 19880-0400(US)**

(72) Inventor: **Brzeczko, Albert Walter**
**408 Grays Lake Way**
**Aberdeen, Maryland 21001(US)**
Inventor: **Nibbelink, Donald Wilfred**
**1 School Road**
**Wilmington, Delaware 19803(US)**
Inventor: **Mollica, Joseph A.**
**P.O. Box 421**
**Montchanin, Delaware 19710(US)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) Deprenyl/L-dopa/carbidopa pharmaceutical composition.

(57) Disclosed are pharmaceutical combination products useful for the treatment of Parkinson's disease. These products are based on a combination of active ingredients which are carbidopa, levodopa, and deprenyl in a pharmaceutical carrier, preferably a carrier wherein one or more of the active ingredients are in a sustained release oral dosage formulation.

EP 0 451 484 A1

## BACKGROUND OF THE INVENTION

Field of the Invention:

This invention relates to combination pharmaceutical compositions for and methods of treating Parkinson's disease and more particularly to such compositions containing and such methods using a combination of carbidopa, levodopa, and deprenyl.

Background Information:

The combination of carbidopa and levodopa has been the treatment of choice for the alleviation of the signs and symptoms of Parkinson's disease. All patients suffering from Parkinson's disease require levodopa therapy at some time during the course of their illness in order to maintain a sufficient amount of dopamine in the brain. Dopamine deficiency in the basal ganglia is the primary reason for the inability to maintain proper motor function for routine daily activities of Parkinsonian patients. Normal motor activity of voluntary and involuntary movements are required for maintenance of all usual activities of daily living.

Sinemet® (Merck & Co., Inc., Rahway, NJ) is the registered trademark for the combination of carbidopa and levodopa which is used for the treatment of idiopathic Parkinson's disease. Since 1975, this treatment has been widely used, but long-term treatment has been associated with "wearing-off" and "on-off" phenomena. This problem usually arises after two to three years of therapy in 15-40% of patients. The rapid fluctuations in levodopa levels which accompany Sinemet® treatment are generally considered to contribute to the cause of fluctuating motor oscillations during the advanced stages of the disease.

A new formulation of carbidopa and levodopa has recently been developed which controls the release characteristics of the tablet as it dissolves in the stomach. This formulation has been designated Sinemet® CR (see U.S. patent 4,832,957, granted May 23, 1989). This formulation has been shown to decrease plasma level fluctuations of levodopa, require less daily dosing frequency than with Sinemet®, and fewer motor fluctuations are reported in patients with advanced disease. Current studies indicate that early treatment of Parkinson's disease with Sinemet® CR significantly alleviates the major motor fluctuations of the disease with an average daily dosage of 400-600 mg daily given in equally divided doses. In this manner, patients derive an improved profile of activity in ordinary living.

Deprenyl has also been used for the treatment of idiopathic Parkinson's disease. Its use in Europe (primarily German speaking countries and the U.K.) has shown partial alleviation of the progression of symptoms of the disease when given alone. Recent studies suggest that it slows the progression of early Parkinson's signs and symptoms, but the cardinal symptoms of rigidity, tremor, bradykinesia and postural instability continue to require treatment. Deprenyl is described in U.S. patent 3,496,195, granted February 17, 1970, and U.K. Patent 1,142,981.

These considerations indicate that a formulation of deprenyl with Sinemet® CR may provide the optimum therapy for idiopathic Parkinson's disease. The basic reason for this proposal is derived from the observation that deprenyl slows the progression of the disease while Sinemet® CR maintains maximum alleviation of signs and symptoms without development of wide fluctuations in levodopa plasma levels or motor oscillations. This treatment has the potential for providing longer life expectancy, improved quality of life, better sociability, and less required medical care. In addition, the advanced forms of the disease may be prevented and fewer complications of therapy may occur as a result due to lower and less frequent doses of levodopa required during the early stages of the disease.

Summary of the Invention

According to the present invention there are provided oral pharmaceutical compositions (tablets or capsules) which comprise a suitable pharmaceutical carrier and a combination of active ingredients consisting essentially of an effective amount of carbidopa, levodopa, and deprenyl.

Also provided is a method of treating Parkinson's disease in a mammal by administering to the mammal concomitantly an effective amount of carbidopa, levodopa, and deprenyl.

Detailed Description of the Invention

The present invention is a pharmaceutical composition comprising effective amounts of the active ingredients carbidopa, levodopa, and deprenyl. The present invention is also a method of treating Parkinson's disease in a mammal by the concomitant administration of effective amounts of the above-mentioned active ingredients. Typically the compositions will contain the active ingredients in the amounts of about 25-100 mg of carbidopa, 100-400 mg of levodopa, and 2-5 mg of deprenyl. In preferred embodiments, about 50 mg of carbidopa, about 200 mg of levodopa, and about 2 mg of deprenyl will be contained in an oral dosage formulation wherein one or more of the active ingredients may be in sustained or controlled release form.

The concomitant administration of a combination of carbidopa, levodopa, and deprenyl uses the deprenyl to decrease the intracerebral metabolic degradation of dopamine which is produced in the brain by the carbidopa and levodopa; thus, less levodopa is needed. Such administration, particularly when combined in a single combination formulation, provides several advantages in the treatment of Parkinson's disease. First, the side effects of levodopa should be minimized because a lower dose can be administered over time. Second, by administering the combination early in the disease, the progression of the disease may be minimized so as to provide a higher quality of life to the patient. This in turn leads to better individual sociability, a longer productive life, reduced medical care, and improved life expectancy. Third, increased safety to the patient should result due to diminished dyskinesia or other abnormal involuntary movements.

The active ingredients used in the combination products of this invention are well known to those in pharmaceuticals. As stated earlier, deprenyl is described in U.S. Patent 3,496,195 and U.K. Patent 1,142,981. Carbidopa and levodopa in combination are described in U.S. Patent 4,832,957. Carbidopa and levodopa are described in The Merck Index, Ninth Edition, 1976, at 1800 and 5310, respectively.

The formulations of the present invention may comprise sustained or controlled-release polymers providing for sustained or controlled release of the active ingredients individually or any combination thereof. Preferably, the formulations provide the sustained-release of levodopa and/or carbidopa and the immediate release of deprenyl.

Polymers useful for sustained release of the active ingredients include but are not limited to drug-release retarding agents such as vinyl acetate-crotonic acid copolymer, hydroxypropyl cellulose, methacrylic acid copolymer, hydroxypropyl methyl cellulose, ethyl cellulose, hydroxyethyl cellulose or a combination thereof. Preferably, a combination of a "water-permeable" polymer and a "less water-permeable" polymer is used such that the water-permeable polymer will disperse drug more quickly than the less water-permeable polymer. Specifically preferred is a combination of hydroxypropyl cellulose (water-permeable) in an amount of about 5-25 mg/unit dose and vinyl acetate-crotonic acid copolymer (less water-permeable) in an amount of about 2-50 mg/unit dose.

The combination products of the present invention can be in any dosage form known to those skilled in the art, such as tablets either single or multi-layered, capsules, caplets, liquids, or suppositories. The preferred dosage forms are tablets, capsules, and caplets in controlled or sustained release formulations. These preferred dosage forms can be made by methods known to those skilled in the art and described in Remington's Pharmaceutical Sciences, (1985), 17th Edition, Osol, a standard reference in the field.

The formulations of the present invention may comprise, in addition to active ingredients, appropriate excipients, including but not limited to: lubricants such as magnesium stearate, stearic acid, sodium stearyl fumarate, and hydrogenated vegetable oil; diluents such as lactose, microcrystalline cellulose, dextrose, starch and dicalcium phosphate; a glidant such as colloidal silicon dioxide; disintegrates such as sodium starch glycolate, croscarmellose sodium and crospovidone, and a wet binder such as povidone. The use of such excipients is known to those skilled in the art of pharmaceutical formulations.

The following examples describe various formulations of levodopa, carbidopa and deprenyl combination products. These formulations are merely exemplary of many such formulations which can be made, it being understood that those skilled in the art can make obvious modifications thereof.

EXAMPLE 1

Dispersing Bead Tablet Containing
Levodopa, Carbidopa and Deprenyl

| Ingredient | Amount (mg) per Dosage Unit |
|---|---|
| Levodopa, USP | 200 |
| Carbidopa Hydrous, USP | 50 |
| Deprenyl | 5 |
| Vinyl Acetate-Crotonic Acid Copolymer | 10 |
| Hydroxypropyl Cellulose, NF | 20 |
| Microcrystalline Cellulose, NF | 100 |
| Crospovidone, NF | 11 |
| Magnesium Stearate, NF | 4 |
| | To total 400 mg |

Prior to weighing pass all ingredients through an appropriate screen as needed, weigh all ingredients and granulate, by methods known to those skilled in the art, the levodopa and carbidopa with retarding agents (hydroxypropyl cellulose and vinyl acetate-crotonic acid copolymer) and optional excipients as needed, into spherical beads. Blend the levodopa and carbidopa sustained release beads with a granulation of immediate release deprenyl. The resulting blend is then compressed into a tablet using an appropriate tableting machine such that the sustained release levodopa and carbidopa beads are entrapped in an immediate release deprenyl tablet.

EXAMPLE 2

Matrix Tablet or Capsule Containing
Levodopa, Carbidopa and Deprenyl

| Ingredient | Amount (mg) per Dosage Unit |
|---|---|
| Levodopa, USP | 200 |
| Carbidopa Hydrous, USP | 50 |
| Deprenyl | 5 |
| Vinyl Acetate-Crotonic Acid Copolymer | 10 |
| Hydroxypropyl Cellulose, NF | 20 |
| Povidone, NF | 10 |
| Magnesium Stearate, NF | 5 |
| | To total 300 mg |

In this formulation, a sustained release matrix tablet containing carbidopa and levodopa is first manufactured by methods known in the art, and this tablet is either placed in a capsule along with a deprenyl granulation and excipients as needed, or the matrix tablet is layer coated, by methods known to those skilled in the art, with immediate release deprenyl.

The sustained release matrix tablet is produced by granulating the levodopa and carbidopa and optional excipients as needed with appropriate retarding agents (vinyl acetate-crotonic acid copolymer and hydroxypropyl cellulose) by methods known in the art. This matrix tablet is then either additionally layer coated with deprenyl and optionally additional excipients as needed or is placed in an appropriate capsule along with an immediate release deprenyl granulation optionally containing excipients as needed.

## EXAMPLE 3

### Dispersing Bead Tablet Containing Levodopa, Carbidopa and Deprenyl

| Ingredient | Amount (mg) per Dosage Unit |
|---|---|
| Levodopa, USP | 200 |
| Carbidopa Hydrous, USP | 50 |
| Deprenyl | 2 |
| Methacrylic Acid Copolymer, NF | 40 |
| Povidone, NF | 10 |
| Microcrystalline Cellulose, NF | 80 |
| Crospovidone, NF | 14 |
| Calcium Stearate, NF | 4 |
| | To total 400 mg |

A tablet is made by the method described in Example 1 such that the sustained release levodopa and carbidopa beads are entrapped in an immediate release deprenyl tablet.

## EXAMPLE 4

### Matrix Tablet or Capsule Containing Levodopa, Carbidopa and Deprenyl

| Ingredient | Amount (mg) per Dosage Unit |
|---|---|
| Levodopa, USP | 200 |
| Carbidopa Hydrous, USP | 50 |
| Deprenyl | 2 |
| Methacrylic Acid Copolymer, NF | 30 |
| Povidone, NF | 13 |
| Calcium Stearate, NF | 5 |
| | To total 300 mg |

A tablet or capsule is made by the method described in Example 2 whereby a matrix tablet containing carbidopa and levodopa is either additionally layer coated with immediate release deprenyl and optional excipients as needed, or is placed in a capsule along with an immediate release deprenyl granulation optionally containing additional excipients.

EXAMPLE 5

**Dispersing Bead Tablet Containing
Levodopa, Carbidopa and Deprenyl**

| Ingredient | Amount (mg) per Dosage Unit |
|---|---|
| Levodopa, USP | 100 |
| Carbidopa Hydrous, USP | 100 |
| Deprenyl | 2 |
| Hydroxypropylmethyl Cellulose, NF | 30 |
| Ethyl Cellulose, NF | 45 |
| Microcrystalline Cellulose, NF | 100 |
| Crospovidone, NF | 20 |
| Magnesium Stearate, NF | 3 |
| | To total 400 mg |

A tablet is made by the method described in Examples 1 and 3 except that the retarding agents are hydroxypropylmethyl cellulose and ethyl cellulose.

EXAMPLE 6

**Matrix Tablet or Capsule Containing
Levodopa, Carbidopa and Deprenyl**

| Ingredient | Amount (mg) per Dosage Unit |
|---|---|
| Levodopa, USP | 100 |
| Carbidopa Hydrous, USP | 100 |
| Deprenyl | 2 |
| Hydroxypropylmethyl Cellulose, NF | 50 |
| Ethyl Cellulose, NF | 45 |
| Magnesium Stearate, NF | 3 |
| | To total 300 mg |

A tablet or capsule is made by the method described in Examples 2 and 4 except that the retarding agents are hydroxypropylmethyl cellulose and ethyl cellulose.

EXAMPLE 7

### Dispersing Bead Tablet Containing
### Levodopa, Carbidopa and Deprenyl

| Ingredient | Amount (mg) per Dosage Unit |
|---|---|
| Levodopa, USP | 400 |
| Carbidopa Hydrous, USP | 25 |
| Deprenyl | 2 |
| Ethyl Cellulose | 30 |
| Hydroxyethyl Cellulose, NF | 30 |
| Microcrystalline Cellulose, NF | 100 |
| Crospovidone, NF | 10 |
| Magnesium Stearate, NF | 3 |
| | To total 600 mg |

A tablet is made by the method described in Examples 1, 3 and 5 except that the retarding agents are ethyl cellulose and hydroxyethyl cellulose.

EXAMPLE 8

### Matrix Tablet or Capsule Containing
### Levodopa, Carbidopa and Deprenyl

| Ingredient | Amount (mg) per Dosage Unit |
|---|---|
| Levodopa, USP | 400 |
| Carbidopa Hydrous, USP | 25 |
| Deprenyl | 2 |
| Ethyl Cellulose | 30 |
| Hydroxyethyl Cellulose, NF | 30 |
| Povidone, NF | 8 |
| Magnesium Stearate, NF | 5 |
| | To total 500 mg |

A tablet or capsule is made by the method described in Examples 2, 4 and 6 except that the retarding agents are ethyl cellulose and hydroxyethyl cellulose.

**Claims**

1. An oral pharmaceutical composition comprising a suitable pharmaceutical carrier and a combination of active ingredients consisting essentially of an effective amount of carbidopa, levodopa and deprenyl.

2. The pharmaceutical composition of Claim 1 wherein the active ingredients are delivered in the amounts of about 25-100 mg of carbidopa, about 100-400 mg of levodopa, and about 2-5 mg of deprenyl.

3. The pharmaceutical composition of Claim 2 wherein the pharmaceutical carrier provides a sustained-release oral dosage composition for one or more of the active ingredients.

4. The pharmaceutical composition of Claim 3 wherein the pharmaceutical carrier provides a sustained-release oral dosage composition for levodopa.

5. The pharmaceutical composition of Claim 3 wherein the pharmaceutical carrier provides a sustained-release oral dosage composition for carbidopa.

6. The pharmaceutical composition of Claim 3 wherein the pharmaceutical carrier provides a sustained-release oral dosage composition for levodopa and carbidopa.

7. The pharmaceutical composition of Claim 3 wherein the sustained-release pharmaceutical carrier is a polymeric vehicle comprising a combination of a water-permeable polymer and a less water-permeable polymer.

8. The pharmaceutical composition of Claim 4 wherein the sustained-release pharmaceutical carrier is a polymeric vehicle comprising a combination of a water-permeable polymer and a less water-permeable polymer.

9. The pharmaceutical composition of Claim 5 wherein the sustained-release pharmaceutical carrier is a polymeric vehicle comprising a combination of a water-permeable polymer and a less water-permeable polymer.

10. The pharmaceutical composition of Claim 6 wherein the sustained-release pharmaceutical carrier is a polymeric vehicle comprising a combination of a water-permeable polymer and a less water-permeable polymer.

11. The pharmaceutical composition of Claim 7 wherein the sustained-release pharmaceutical carrier is a polymeric vehicle comprising about 5-25 mg/unit dose of a water-permeable polymer and about 2-50 mg/unit dose of a less water-permeable polymer.

12. The pharmaceutical composition of Claim 8 wherein the sustained-release pharmaceutical carrier is a polymeric vehicle comprising about 5-25 mg/unit dose of a water-permeable polymer and about 2-50 mg/unit dose of a less water-permeable polymer.

13. The pharmaceutical composition of Claim 9 wherein the sustained-release pharmaceutical carrier is a polymeric vehicle comprising about 5-25 mg/unit dose of a water-permeable polymer and about 2-50 mg/unit dose of a less water-permeable polymer.

14. The pharmaceutical composition of Claim 10 wherein the sustained-release pharmaceutical carrier is a polymeric vehicle comprising about 5-25 mg/unit dose of a water-permeable polymer and about 2-50 mg/unit dose of a less water-permeable polymer.

15. The pharmaceutical composition of Claim 11 wherein the water-permeable polymer is a hydroxypropyl cellulose polymer and the less water-permeable polymer is a polyvinyl acetate-crotonic acid copolymer.

16. The pharmaceutical composition of Claim 12 wherein the water-permeable polymer is a hydroxypropyl cellulose polymer and the less water-permeable polymer is a polyvinyl acetate-crotonic acid copolymer.

17. The pharmaceutical composition of Claim 13 wherein the water-permeable polymer is a hydroxypropyl cellulose polymer and the less water-permeable polymer is a polyvinyl acetate-crotonic acid copolymer.

18. The pharmaceutical composition of Claim 14 wherein the water-permeable polymer is a hydroxypropyl cellulose polymer and the less water-permeable polymer is a polyvinyl acetate-crotonic acid copolymer.

19. The pharmaceutical composition of Claim 15 wherein the active ingredients consist essentially of about 50 mg/unit dose of caridopa, about 200 mg/unit dose of levodopa and about 2 mg/unit dose of deprenyl.

8

20. The pharmaceutical composition of Claim 16 wherein the active ingredients consist essentially of about 50 mg/unit dose of caridopa, about 200 mg/unit dose of levodopa and about 2 mg/unit dose of deprenyl.

21. The pharmaceutical composition of Claim 17 wherein the active ingredients consist essentially of about 50 mg/unit dose of caridopa, about 200 mg/unit dose of levodopa and about 2 mg/unit dose of deprenyl.

22. The pharmaceutical composition of Claim 18 wherein the active ingredients consist essentially of about 50 mg/unit dose of caridopa, about 200 mg/unit dose of levodopa and about 2 mg/unit dose of deprenyl.

23. A method of treating Parkinson's disease in a mammal comprising administering to the mammal concomitantly an effective amount of levodopa, carbidopa and deprenyl.

24. A method of treating Parkinson's disease in a mammal comprising administering to the mammal an effective amount of any of the pharmaceutical compositions of Claims 1-22.

European Patent Office

# PARTIAL EUROPEAN SEARCH REPORT

which under Rule 45 of the European Patent Convention shall be considered, for the purposes of subsequent proceedings, as the European search report

**Application number**

EP 91 10 2994

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DIALOG 7034630, EMBASE (EXERPTA MEDICA) abstr. no. 880349433; L.I. GOLBE et al.: "Deprenyl in the treatment of symptom fluctuations in advanced Parkinson's disease", & CLIN. NEUROLPHARMACOL. (USA), 1988, 11/1 (45-55) | | A 61 K 31/195// A 61 K 31/195 A 61 K 31/14 |
| | * Abstract * | 1 | |
| Y | | 2-18 | |
| | -- | | |
| Y | EP-A-0 320 051 (MERCK & CO. INC.) | | |
| | * The whole document * | 2-18 | |
| | -- | | |
| X | DIALOG 5560317, EMBASE (EXERPTA MEDICA) abstr. no. 880349433; A.N. LIEBERMAN et al.: "Deprenyl in the treatment of Parkinson's ./. | | TECHNICAL FIELDS SEARCHED (Int. Cl.4)  A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-22
Claims searched Incompletely:
Claims not searched: 23,24
Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 04-06-1991 | LEHERTE |

EPO Form 1505.1 03 82

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| | disease". A specific type B mono-amine oxidase inhibitor. N.Y. STATE J. MED. (USA), 1984, 84./1 (13-16)<br><br>* Abstract * | 1 | |
| Y | | 2-18 | |
| | -- | | |
| Y | EP-A-0 253.490 (MERCK & CO. INC)<br><br>* The whole document * | 2-18 | TECHNICAL FIELDS SEARCHED (Int Cl.4) |
| | -- | | |
| X | BIOLOGICAL ABSTRACTS, vol. 89, 1990, abstract no. 52183, Philadelphia, PA, US; T.S. ELIZAN et al.: "Selegiline use to prevent progression of parkinson's disease experience in 22 de novo patients", & ARCH. NEUROL. 1989, 46 (12): 1275-1279<br><br>* Abstract * | 1 | |
| | -- | | |
| X | BIOLOGICAL ABSTRACTS, vol. 85, 1988, abstract no. 61368, Philadelphia, PA, US; A.N. LIEBERMAN et al.: "Deprenyl versus placebo in Parkinson disease a double-blind study", & N.Y. STATE, J. MED. 1987, 87(12) 646-649<br><br>* Abstract * | 1 | |
| | ---- | | |